(19) Europäisches Patentamt

European Patent Office

Office européen des brevets

(11) Veröffentlichungsnummer: **0 254 929 B1**

(12) **EUROPÄISCHE PATENTSCHRIFT**

(45) Veröffentlichungstag der Patentschrift: **25.03.92**

(21) Anmeldenummer: **87109953.7**

(22) Anmeldetag: **10.07.87**

(51) Int. Cl.⁵: **G01N  33/543**, G01N 33/52, //G01N33/94,G01N33/74, G01N33/92

(54) **Verfahren zum Nachweis eines Analyten sowie hierfür geeignetes Mittel.**

(30) Priorität: **19.07.86 DE 3624464**

(43) Veröffentlichungstag der Anmeldung: **03.02.88 Patentblatt  88/05**

(45) Bekanntmachung des Hinweises auf die Patenterteilung: **25.03.92 Patentblatt  92/13**

(84) Benannte Vertragsstaaten: **AT BE CH DE ES FR GB GR IT LI LU NL SE**

(56) Entgegenhaltungen:
EP-A- 26 103   EP-A- 89 806
EP-A- 106 615   EP-A- 143 274
EP-A- 182 385   EP-A- 257 352
WO-A-83/03306   WO-A-85/00226
DE-A- 3 227 474

(73) Patentinhaber: **BOEHRINGER MANNHEIM GMBH**
**Sandhofer Strasse 116**
**W-6800 Mannheim 31(DE)**

(72) Erfinder: **Wilk, Hans-Erich, Dr.**
**Goldregenstr. 5**
**W-6143 Lorsch(DE)**
Erfinder: **Freitag, Helmut, Dr.**
**An der Ziegelhütte 13**
**W-6940 Weinheim(DE)**
Erfinder: **Burg, Josef, Dr.**
**Hackerberg 4**
**W-8033 Krailling(DE)**
Erfinder: **Berger, Johann, Dr.**
**9115 Hague Road**
**Indianapolis, IN 46250(US)**

## Beschreibung

Die vorliegende Erfindung betrifft ein Verfahren zum Nachweis eines Analyten in einer Probe sowie ein für dieses Verfahren geeignetes Mittel.

Immunologische Nachweisverfahren spielen seit mehreren Jahren eine wesentliche Rolle in der klinischen Diagnostik. Sie zeichnen sich dadurch aus, daß sie hochspezifisch und äußerst empfindlich sind. Diese Nachweisverfahren basieren auf der immunologischen Wechselwirkung zwischen dem nachzuweisenden Analyten und seinem Bindungspartner bzw. -partnern. Durch Markieren eines der Bindungspartner kann der Grad der Umsetzung und damit die Konzentration des zu messenden Analyten bestimmt werden. Die immunologischen Bestimmungsverfahren werden je nach der gewählten Markierung unterschieden, beispielsweise Radioimmunoassay, (radioaktive Markierung), Enzymimmunoassay, (Enzymmarkierung), Fluoreszenzimmunoassay (Fluoreszenzmarker) usw.

Nachteilig für diese Methoden ist, daß teilweise sehr lange Inkubationszeiten erforderlich sind. Die Nachweisverfahren sind sehr arbeitsintensiv. Bei den Radioimmunoassays kommen die Schwierigkeiten hinzu, die mit der Handhabung radioaktiver Substanzen verbunden sind.

Es hat daher nicht an Versuchen gefehlt, diese Teste zu verbessern. So läuft beispielsweise ein Meßverfahren, das für viele Anwendungszwecke besonders bevorzugt ist, derart ab, daß der zu messende Analyt zunächst mit dem markierten Bindungspartner im Überschuß inkubiert wird. Der vom Analyten nicht gebundene markierte Bindungspartner wird mit Hilfe von trägergebundenem Analyten fixiert. Nach einer üblichen Trennung des gebundenen vom nicht gebundenen Anteil kann anhand der Markierung der ursprüngliche Analytgehalt der Probe bestimmt werden.

Dieses Meßprinzip weist wie alle übrigen immunologischen Verfahren eine sehr hohe Empfindlichkeit auf, hat aber darüber hinaus den Vorteil, daß es schneller durchgeführt werden kann als die bisher üblichen Testführungen. Ein wesentlicher Nachteil dieser Variante ist jedoch, daß der markierte Bindungspartner des zu bestimmenden Analyten im Überschuß eingesetzt werden muß, d. h. es werden relativ große Mengen an markierten immunologischen Bindungspartnern benötigt.

In der EP-A-0 143 274 wird beispielsweise ein Immunoassay zum Nachweis von Digoxin beschrieben. Die Sensitivität des Testes ist erhöht, da als trägergebundener Analyt nicht Digoxin, sondern Ouabain eingesetzt wird, zu dem der markierte Antikörper eine niedrigere Affinität besitzt. Der Antikörper muß im Überschuß zu Digoxin eingesetzt werden.

Der Nachweis von hochkonzentrierten Analyten in unverdünntem Blut, Serum oder Plasma ist wegen der erforderlichen hohen Konzentration an Antikörper und der damit verbundenen Kosten kaum mehr durchführbar.

In der EP-A-0 182 385 wird ein Verfahren zur Bestimmung von freiem Thyroxin beschrieben. Durch die Kombination einer sehr kurzen Inkubationszeit und eines Unterschusses an markiertem Anti-$T_4$-Antikörpers mit hoher Affnität zu $T_4$ gelingt es, die außerordentlich geringen Mengen an freiem $T_4$ genau zu bestimmen. Ein Verfahren zur Bestimmung von hochkonzentriertem Analyten in einer Probe wird nicht offenbart.

Die WO 85/00226 beinhaltet ein Verfahren zur Konzentrationsbestimmung von freien Liganden. Zusätzlich zum spezifischen Bindepartner zum freien Liganden und einem Ligandenanalogon enthält der Testansatz einen zweiten, exogenen nichtmarkierten Bindepartner, der das Ligandenanalogon bindet. Dieser zusätzliche Bindepartner dient zur Beseitigung der Störungen, die durch die mögliche Bindung des Ligandenanalogon an die endogenen, in der Probe vorhandenen Bindeproteine auftreten.

In der WO 83/03306 wird ebenfalls ein Verfahren zur Bestimmung von sehr niedrig konzentrierten freien Liganden beschrieben, das ein nichtmarkiertes Ligandenanalogon und einen Ligandenrezeptor nutzt. Das Ligandenanalogon soll nicht an die endogenen Rezeptoren binden, aber vergleichbar gut dem Liganden an den Ligandenrezeptor.

Das Verfahren der EP-A-0 089 806 zum Nachweis von freien Analyten verlangt, daß der markierte spezifische Bindepartner des Analyten in einer Menge eingesetzt wird, die das Gleichgewicht zwischen dem gebundenen und freien Analyten nicht stört. Ein Verfahren zur Verminderung der Menge an markiertem Bindungspartner zur Bestimmung von hochkonzentrierten Analyten wird nicht beschrieben.

Die EP-A-0 026 103 beschreibt ebenfalls ein Verfahren zur Bestimmung von freien Liganden. Hier wird ein markiertes Ligandenanalogon und ein unmarkierter spezifischer Bindepartner des Liganden eingesetzt. Dieses Verfahren dient zum Nachweis von sehr niedrig konzentrierten freien Liganden. Ein Nachweis von hochkonzentrierten Analyten wird in keinem dieser Verfahren zur Bestimmung von freien Liganden angesprochen.

Aufgabe der Erfindung war es daher, ein Verfahren bereitzustellen, bei dem nicht so hohe Konzentrationen an markierten Bindungspartnern erforderlich sind und das daher kostengünstiger als die bisher bekannten Verfahren durchgeführt werden kann. Diese Aufgabe wird durch das beanspruchte Verfahren gelöst.

Gegenstand der Erfindung ist ein Verfahren zum Nachweis eines Analyten, dadurch gekennzeichnet, daß

- die den Analyten enthaltende Probe entweder

mit einem markierten Bindungspartner und dem immobilisierten Analyten oder einem immobilisierten Analyt-Analogon

oder

mit dem markierten Analyten oder Analyt-Analogon und einem immobilisierten Bindungspartner inkubiert wird,

- der Bindungspartner gegenüber dem immobilisierten bzw. markierten Analyten oder Analyt-Analogon eine höhere Affinität aufweist als gegenüber dem freien Analyten und
- der markierte Bindungspartner bzw. der markierte Analyt oder das markierte Analyt-Analogon im Unterschuß verglichen mit der Konzentration des nachzuweisenden Analyten vorliegen.

Unter einem Analyten wird ganz allgemein eine Substanz verstanden, die mit einem geeigneten Bindungspartner eine spezifische Wechselwirkung eingehen kann. Hierunter wird beispielsweise jede Art von Wechselwirkung verstanden, z. B. eine Antigen-Antikörper-, Enzym-Coenzym- oder auch irgendeine sonstige Ligand-Rezeptor-Wechselwirkung. Der Analyt kann vorzugsweise ein Hapten, z. B. Theophyllin, Thyroxin (T4), Phenobarbital usw., ein Antigen, z. B. eine Nucleinsäure oder ein Protein, wie humanes Choriogonadotropin (hCG), Human-Serum-Albumin (HSA), Carcinoembrionales Antigen (CEA), $\alpha$-Foetoprotein (AFP), glycosiliertes Hämoglobin (HbA$_1$), Immunglobuline usw., oder auch ein Antikörper sein. Der Bindungspartner wird durch den jeweiligen Analyten bestimmt. Ist der Analyt beispielsweise ein Hapten oder Antigen, so wird als Bindungspartner ein gegen dieses Hapten oder Antigen gerichteter Antikörper benötigt; soll ein Antikörper bestimmt werden, so ist als Bindungspartner ein zugehöriges Antigen oder Hapten oder ein Anti-Antikörper erforderlich.

Unter einem Analyt-Analogon wird im Sinne der vorliegenden Erfindung eine Verbindung verstanden, die eine strukturelle Verwandtschaft zum Analyten und somit auch verwandte Bindungseigenschaften aufweist. Solche Analyt-/ Analyt-Analogon-Paare sind beispielsweise T3/T4, Theophyllin/Theobromin, Human-Serum-Albumin/Affen-Serum-Albumin. Als ein Analyt-Analoga sind auch anti-idiotypische Antikörper einsetzbar, die gegen die Antigenbindungsstelle gerichtet sind. Solche Antikörper haben zwangsläufig eine strukturelle Verwandtschaft mit dem zu bestimmenden Analyten.

Unter Antikörper im Sinne der Erfindung sind sowohl monoklonale als auch polyklonale Antikörper zu verstehen. Sie können als vollständige Antikörper oder auch in Form von Antikörper-Fragmenten, beispielsweise Fab-Fragmenten, eingesetzt werden. Erfindungsgemäß eingesetzt werden Antikörper, die den trägergebundenen bzw. markierten Analyten bzw. das trägergebundene bzw. markierte Analyt-Analogon spezifischer erkennen bzw. fester binden als den freien Analyten.

Solche Antikörper sind beispielsweise Antikörper, die das Brückenglied miterkennen, mit dem der Analyt bzw. das Analyt-Analogon an die Trägermatrix bzw. an das Markierungsmittel gebunden ist. Derartige Antikörper können nach bekannten Methoden gewonnen werden, wobei zur Immunisierung eine Substanz verwendet wird, die aus dem Analyten bzw. dem Analyt-Analogon, dem Brückenglied sowie, falls der Analyt bzw. das Analyt-Analogon nicht selbst immunogen wirksam ist, einem immunogenen Protein, z. B. Albumin, Edestin usw., besteht.

Es können auch Antikörper eingesetzt werden, die das Analyt-Analogon spezifisch erkennen und mit dem eigentlichen Analyten nur niedrigaffin kreuzreagieren. So kann beispielsweise nach dem erfindungsgemäßen Verfahren T4 bestimmt werden, wenn an die Trägermatrix T3 gebunden wird und ein Antikörper benutzt wird, der hochaffin gegenüber T3, jedoch nur wenig affin gegenüber T4 ist. Auf analoge Weise lassen sich auch Proteine bestimmen. So läßt sich in erfindungsgemäßer Weise Human-Serum-Albumin (HSA) nachweisen, indem Affen-Serum-Albumin an die Festphase gebunden wird und der Antikörper gegen das Affen-Serum-Albumin gerichtet ist mit geringer Kreuzreaktivität zu Humanserum-Albumin. Der Analyt kann auch bestimmt werden, wenn man als Analyt-Analogon eine bestimmte Teilstruktur oder Teilsequenz an die Trägermatrix bindet und einen Antikörper einsetzt, der diese immobilisierte Teilstruktur oder Teilsequenz spezifischer erkennt als den nachzuweisenden Analyten. Diese Variante läßt sich z. B. vorteilhaft für einen Nachweis des glycosilierten Hämoglobins ausnützen, indem an die Festphase ein synthetisches Peptid mit der glycosilierten Stelle des glycosilierten Hämoglobins gebunden ist und ein Antikörper gegen dieses Peptid eingesetzt wird.

Mit Hilfe solcher Antikörper ist es möglich, in dem erfindungsgemäßen Verfahren den enzymmarkierten Bindungspartner bzw. den markierten Analyten oder das markierte Analyt-Analogon in einem Unterschuß gegenüber dem zu bestimmenden Analyten einzusetzen. Wie verschieden die Konzentrationen von nachzuweisendem Analyt und markiertem Bindungspartner bzw. markiertem Analyten oder Analyt-Analogon sein können, ist entscheidend davon abhängig, wie groß der Affinitätsunterschied des Bindungspartners gegenüber dem freien Analyten und dem immobilisierten bzw. markierten Analyten oder Analyt-Analogon ist. Je niedrigeraffin der Bindungspartner gegenüber dem freien Analyten verglichen mit dem gebundenen bzw. markierten Analyten oder Analyt-Analogon ist, desto niedriger kann die Konzentration des markierten Bindungspartner bzw. des markierten Analyten oder Analyt-Analogon im Vergleich zur Konzentration des zu bestimmenden Analyten sein. Es hat sich gezeigt, daß das erfindungsgemäße Verfahren bei einem Konzentrationsverhältnis von markiertem Bindungspartner bzw. markiertem Analyten oder Analyt-Analogon zu nachzuweisendem Analyten bis 1:10000 mit Vorteil durchzuführen ist, wenn der eingesetzte Bindungspartner einen Affinitätsunterschied gleicher Größenordnung aufweist. Besonders vorteilhaft hat sich ein Konzentrationsverhältnis von 1:2 bis 1:3000 gezeigt.

Bei der Inkubation der Probe, die den zu bestimmenden Analyten enthält, mit dem markierten Bindungspartner und dem trägergebundenen Analyten bzw. Analyt-Analogon konkurrieren im wesentlichen die folgenden Reaktionen miteinander:

(1)    $A + B\text{-}M \rightleftarrows A\text{-}B\text{-}M$

(2)    $A\text{-}B\text{-}M + A_{fix} \rightleftarrows A_{fix}\text{-}B\text{-}M + A$

In diesen Gleichungen bedeutet A den zu messenden Analyten, B den gegen den Analyten gerichteten Bindungspartner, M das Markierungsmittel und $A_{fix}$ den an einen Träger gebundenen Analyten bzw. Analyt-Analogon.

Gemäß Gleichung (1) bildet sich aus dem Analyten und dem markierten Bindungspartner B-M der immunologische Komplex A-B-M. Wegen des großen Überschusses des Analyten ist dieses Gleichgewicht weitgehend auf die Seite des markierten immunologischen Komplexes verschoben, so daß im Reaktionsgemisch fast ausschließlich Komplex und überschüssiger freier Analyt vorliegen. Wegen der höheren Affinität des Bindungspartners gegenüber dem gebundenen Analyten $A_{fix}$ findet eine Verdrängungsreaktion gemäß Gleichung (2) statt. Je höher die Konzentration des nachzuweisenden Analyten in der Probe ist, desto stärker wird der Verdrängung des nachzuweisenden Analyten A aus dem immunologischen Komplex A-B-M durch den trägergebundenen Analyten bzw. Analyt-Analogon $A_{fix}$ entgegengewirkt. Die Konzentration des Analyten in der Probe ist demnach direkt proportional der Menge der Markierung im löslichen Teil des Reaktionsgemisches bzw. umgekehrt proportional der Menge der Markierung, die über $A_{fix}$ an den Träger gebunden ist. Durch Messen der an den Träger gebundenen Markierung oder auch der in der Lösung befindlichen Markierung läßt sich auf die Konzentration des Analyten in der Probe schließen.

Analoge Gleichungen können formuliert und analoge Überlegungen können angestellt werden für den Fall, daß die Probe mit dem markierten Analyten bzw. einem markierten Analyt-Analogon und einem immobilisierten Bindungspartner inkubiert wird.

Das erfindungsgemäße Verfahren kann in vorteilhafter Weise derart durchgeführt werden, daß die zu untersuchende Probe mit dem nachzuweisenden Analyten zunächst mit dem markierten Bindungspartner vorinkubiert wird. Dieses Inkubationsgemisch wird anschließend mit der festen Phase an die der Analyt oder ein Analyt-Analogon gebunden ist, in Kontakt gebracht. Es schließt sich eine übliche Trennung des gebundenen von dem nichtgebundenen Anteil an. Als letzter Schritt wird die Markierung im freien oder/und gebundenen Teil bestimmt und hieraus auf den Analyt-Gehalt der Probe geschlossen.

Das Verfahren läßt sich auch derart durchführen, daß die zu untersuchende Probe, der markierte Bindungspartner und der an einen Träger gebundene Analyt bzw. Analyt-Analogon gleichzeitig inkubiert wird. Nach dem Inkubationsschritt wird wiederum der gebundene und der nichtgebundene Anteil getrennt, die Markierung im gebundenen oder/und im nichtgebundenen Anteil bestimmt und hieraus auf die Konzentration des Analyten in der Probe geschlossen.

Ein solches einstufiges Testverfahren kann beispielsweise in Form eines üblichen Mikrotiter-Testes durchgeführt werden. Hierzu wird der Analyt zunächst an der Mikrotiterplatte immobilisiert. Danach wird mit einem Gemisch aus markiertem Bindungspartner und der Probe mit dem zu bestimmenden Analyten inkubiert. Nach einer bestimmten Inkubationszeit wird die Lösung entfernt, es wird gewaschen und anschließend die auf der Mikrotiterplatte gebundene Menge des Markierungsmittels gemessen.

Die vorstehend beschriebenen Verfahrensvarianten lassen sich ohne weiteres auch durchführen, wenn anstelle des markierten Bindungspartners der markierte Analyt oder ein markiertes Analyt-Analogon und anstelle des immobilisierten Analyten bzw. Analyt-Analogons ein immobilisierter Bindungspartner eingesetzt wird.

Die Bindungspartner bzw. der Analyt oder das Analyt-Analogon können in üblicher Weise markiert werden. Dem Fachmann stehen hierzu eine ganze Reihe von bekannten Markierungsmitteln zur Verfügung,

4

beispielsweise die Markierung mit einem radioaktiven Isotop, mit einem Enzym, mit einer fluoreszierenden Substanz oder mit einer anderen Substanz, die photometrisch nachgewiesen werden kann. Im Sinne der vorliegenden Erfindung ist besonders die Markierung mit Enzymen bevorzugt. Als Markierungsenzyme können beispielsweise Peroxidase, alkalische Phosphatase, Glucoseoxidase oder besonders bevorzugt ß-Galactosidase eingesetzt werden.

Die Bindung des Analyten bzw. des Analyt-Analogons oder auch des Bindungspartners an einen Träger erfolgt ebenfalls in einer Weise, die dem Fachmann geläufig ist. Es werden hierzu reaktive Gruppen des Analyten, des Analyt-Analogons bzw. des Bindungspartners ausgenützt. Falls diese Substanzen keine geeigneten reaktiven Gruppen besitzen, können solche nach bekannten Methoden in das Molekül eingeführt werden. Über diese reaktiven Gruppen erfolgt, üblicherweise unter Zwischenschalten eines bifunktionellen Brückengliedes die Verknüpfung mit reaktiven Gruppen des Trägermaterials. Der Bindungspartner kann auch adsorptiv auf den Träger aufgebracht werden. Als Trägermaterialien eignen sich alle üblicherweise für das Immobilisieren von immunologisch wirksamen biochemischen Verbindungen verwendeten Stoffe, z. B. Nitrocellulose, Papiere, Kunststoffpartikel aus Polystyrol o. ä.

Ein weiterer Gegenstand der Erfindung ist ein Mittel zur Durchführung des erfindungsgemäßen Verfahrens. Dieses Mittel ist dadurch gekennzeichnet, daß es

entweder

den immobilisierten Analyten oder ein immobilisiertes Analyt-Analogon und einen markierten Bindungspartner

oder

den markierten Analyten oder ein markiertes Analyt-Analogon und einen immobilisierten Bindungspartner

sowie gegebenenfalls ein geeignetes Puffersystem und weitere zusätzliche, üblicherweise verwendete Hilfsstoffe enthält, wobei der Bindungspartner gegenüber dem immobilisierten bzw. markierten Analyten oder Analyt-Analogon eine höhere Affinität aufweist als gegenüber dem freien Analyten.

Weitere zusätzliche, üblicherweise verwendete Hilfsstoffe sind beispielsweise Netzmittel, Stabilisierungsmittel, galenische Hilfsmittel, Gerüstbildner usw.

Sind zum Nachweis des Markierungsmittels weitere Substanzen erforderlich, so können diese ebenfalls in dem Reagenz enthalten sein. Wird als Markierungsmittel beispielsweise ein Enzym benutzt, so wird das zum Nachweis dieses Enzyms erforderliche Substrat und sonstige Hilfsstoffe vorteilhafterweise dem Mittel beigefügt.

Das Mittel kann auf verschiedenartigste Weise zusammengesetzt sein. Es kann beispielsweise aus verschiedenen Lösungen, die den markierten Bindungspartner bzw. den markierten Analyten oder das markierte Analyt-Analogon, ein geeignetes Puffersystem, Nachweisstoffe für die Bestimmung des Markierungsmittel sowie gegebenenfalls weitere Hilfsstoffe enthalten, und aus einer Festphase, an die der Analyt bzw. das Analyt-Analogon bzw. der Bindungspartner gegebenenfalls über ein Brückenglied gebunden ist, bestehen. Als Lösungsmittel kommen Wasser oder Gemische von Wasser mit einem wasserlöslichen organischen Lösungsmittel, wie z. B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden.

Für bestimmte Ausführungsformen des erfindungsgemäßen Mittels kann es zweckmäßig sein, wenn die Bestandteile des Mittels teilweise oder auch vollständig in lyophilisierter Form vorliegen. Hierzu werden in üblicher Weise zunächst Lösungen der betreffenden Inhaltsstoffe hergestellt und diese in bekannter Weise gefriergetrocknet. Die Lyophilisate werden vor Gebrauch in üblicher Weise mit einem geeigneten Lösungsmittel, z. B. Wasser, rekonstituiert.

Es kann auch zweckmäßig sein, einzelne oder alle Bestandteile des erfindungsgemäßen Mittels in Form von Pulvermischungen oder Reagenztabletten herzustellen. Hierzu werden die Bestandteile des Mittels mit üblichen galenischen Zusatzstoffen versetzt und granuliert. Als solche Zusatzstoffe sind beispielsweise geeignet Kohlenhydrate, wie Mono-, Oligo- oder Polysaccharide, oder Zuckeralkohole, wie Mannit, Sorbit oder Xylit, oder andere inerte Verbindungen, wie Polyethylenglycole oder Polyvinylpyrolidon.

Es ist darüber hinaus möglich, einzelne oder auch alle Bestandteile des Mittels auf einen Träger, insbesondere saugfähigen Träger, aufzubringen bzw. in einen Träger zu inkorporieren. Vorteilhaft kann auch sein, die Reagenzbestandteile auf verschiedene Träger bzw. saugfähige Träger aufzubringen bzw. zu inkorporieren und diese verschiedenen Träger in sinnvoller Weise miteinander zu kombinieren. So ist eine besonders bevorzugte Ausführungsform der erfindungsgemäßen Reagenzes derart aufgebaut, daß auf einem ersten Träger der markierte Bindungspartner enthalten ist, auf einem weiteren Trägermaterial der Analyt bzw. das Analyt-Analogon in immobilisierter Form aufgebracht ist und gegebenenfalls ein dritter Träger die zum Nachweis des Markierungsmittels erforderlichen Reagenzbestandteile enthält. Diese drei Schichten werden so miteinander verbunden, daß die Probe zunächst auf den ersten Träger mit dem markierten Bindungspartner aufgebracht wird. Hier findet dann die Reaktion zwischen dem zu bestimmenden Analyten und dem markierten Bindungspartner statt. Das Reaktionsgemisch wird dann mit der zweiten Schicht in Kontakt gebracht, die den gebundenen Analyten bzw. das gebundene Analyt-Analogon enthält. Dabei findet die Verdrängungsreaktion statt. Der durch den auf den Träger fixierte Analyt bzw. Analyt-Analogon gebundene Anteil des markierten Bindungspartners bleibt auf dieser Trägermatrix haften. Der mit dem zu bestimmenden Analyt verbundene, sogenannte "freie" Anteil des markierten Bindungspartners wird dann auf die dritte Schicht überführt, auf dem der Nachweis des Markierungsmittels, beispielsweise anhand einer Farbreaktion oder photometrisch bestimmt werden kann. Der Transport des Reaktionsgemisches durch die einzelnen Schichten kann mechanisch (z. B. Druck) oder auch durch Diffusion oder Kapillarkräfte erfolgen.

Die Erfindung wird durch die folgenden Beispiele näher erläutert:

Beispiel 1

Nachweis von Theophyllin

A) Herstellung eines polyklonalen Antikörpers gegen Theophyllin-9-carboxypentyl-edestin

Schafe werden in bekannter Weise mit Theophyllin-9-carboxypentyl-edestin immunisiert. Aus den den Schafen entnommenen Antiseren wird in bekannter Weise eine Antikörperlösung isoliert. Die Antikörperlösung wird über Theophyllin-9-pentyl-sepharose aufgereinigt. Die Elution erfolgt mit Theophyllin-Stufengradienten. Die Fraktion, die man mit hoher Theophyllinkonzentration erhält, wird nach Dialyse weiter verwendet.

B) Analytik mit den gewonnenen Antikörpern

Theophyllin-9-carboxypentyl-hydroxysuccinimidester wird an Kaninchen-IgG (unspezifisch) gekoppelt. Die so gewonnenen Polyhaptene (PH) werden mit Bicarbonatpuffer (200 mmolar, pH 9,4) vermischt (10 $\mu$g Polyhapten/ml Carbonatpuffer). Eine Mikrotiterplatte wird mit dieser Polyhaptenlösung beschichtet.

Die in A) beschriebenen Antikörper werden mit Inkubationspuffer (PBS, 1 % Rinderserum-Albumin, 0,1 % TWEEN$^R$ 20) verdünnt, so daß eine $10^{-8}$ M Lösung vorliegt. Ein Volumenteil dieser Lösung wird mit dem gleichen Volumen einer serologischen Verdünnungsreihe (Faktor 1:$2^n$) Theophyllin, Theophyllin-9-carboxypentyl-N-t-BOC-lysin bzw. Coffein versetzt und eine Stunde bei Raumtemperatur inkubiert. N-t-BOC ist eine Abkürzung für den tert.-Butyloxy-carbonyl-Rest, der eine üblicherweise verwendete Stickstoffschutzgruppe vorstellt.

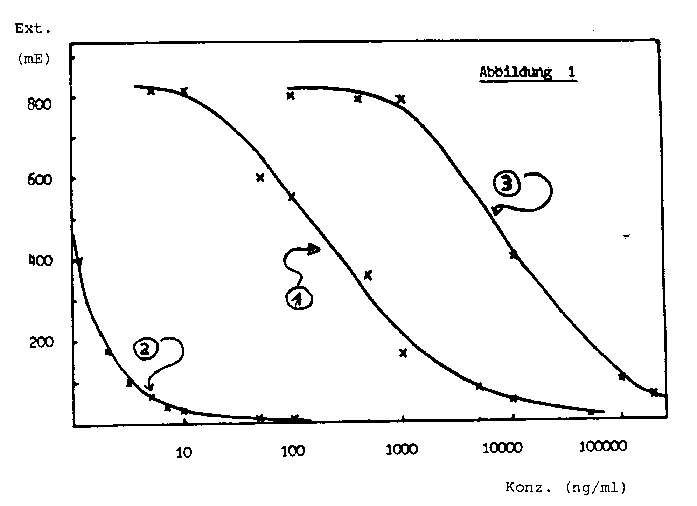

Diese Inkubationsansätze werden in die mit Polyhapten beschichteten Mikrotiterplatten gegeben. Es wird eine weitere Stunde bei Raumtemperatur inkubiert. Danach wird die Lösung entfernt und mehrmals gewaschen. In die Mikrotiterplatte wird schließlich mit Peroxidase-markierter Kaninchen-anti-Schaf-Antikörper (100 mU/ml) gegeben. Es wird eine Stunde bei Raumtemperatur inkubiert und anschließend gewaschen. In jede Vertiefung der Mikrotiterplatte wird 100 $\mu$l ABTS-Substratlösung (ABTS = 2,2'-Azino-d-(3-ethyl-benzthiazolin-6-sulfonsäure)) gegeben. Nach einer Stunde wird mit einem Mikro-ELISA-Reader die Extinktion bestimmt. In Abb. 1 sind typische Meßkurven wiedergegeben. Die Meßkurven gelten
(1) für Theophyllin
(2) für Theophyllin-9-carboxypentyl-N-t-BOC-lysin
(3) für Coffein.
Daraus läßt sich ablesen, daß ein Affinitätsunterschied um den Faktor 200 bis 400 bei einer akzeptablen Kreuzreaktion von 2,5 % mit Coffein besteht.

C) Nachweis von Theophyllin

Der Nachweis von Theophyllin erfolgt völlig analog zu dem in B) angegebenen Verfahren.

20 $\mu$l theophyllinhaltiges Serum und 20 $\mu$l der oben genannten Antikörper-Lösung werden in der unter B) beschriebenen Weise inkubiert und auf einer gemäß B) vorbeschichteten Mikrotiterplatte analysiert. Zunächst wird mit Hilfe von Serum-Proben, die eine genau bekannte Menge, nämlich 2,5, 5, 10, 20 und 40 mg/l Theophyllin enthalten, die folgende Eichtabelle ermittelt.

| $C_{Theo}$ | mE |
|---|---|
| 2,5 | 490 |
| 5 | 392 |
| 10 | 300 |
| 20 | 224 |
| 40 | 143 |

Anhand dieser Tabelle kann der unbekannte Theophyllin-Gehalt in Proben ermittelt werden.

Beispiel 2

Nachweis von Thyroxin (T4)

A) Herstellung eines monoklonalen Antikörpers gegen Tetrajod-Thyronin-N-t-BOC-Edestin

Weibliche Balb/c-Mäuse werden in bekannter Weise mit T4-N-t-BOC-Edestin immunisiert. Milzzellen dieser immunisierter Mäuse werden mit der Myeloma-Zellinie Ag 8.653 nach der bekannten Methode von Köhler und Milstein fusioniert. Gewonnene Klone werden selektioniert. Es werden die Klone ausgewählt, die eine hohe Affinität für T4-N-t-BOC-Immunglobulin besitzen.

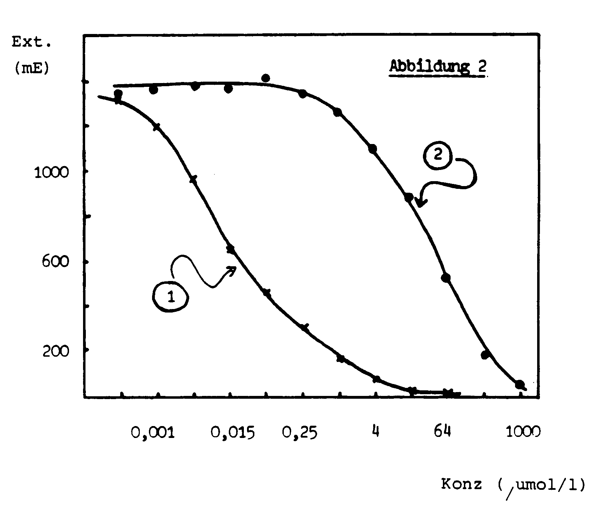

Zur Selektion werden die Antikörper mit einer Konzentrationsreihe von T4 (Faktor $1:4^n$) inkubiert (eine Stunde Raumtemperatur) und danach in einer PH-beschichteten Mikrotiterplatte überführt. Nach weiteren zwei Stunden Inkubation wird die überstehende Lösung entfernt, die Platten gewaschen und analog Beispiel 1 die gebundenen Antikörper mit Peroxidase-markierten anti-Maus-Antikörper und ABTS sichtbar gemacht. Abb. 2 zeigt die Kompetitionskurve eines solchen Antikörpers gegen modifiziertes T4 (Kurve 2) im Vergleich zu einer Kurve, die mit einem anti-T4-Antikörper, der direkt gegen T4 gerichtet ist (Kurve 1), erhalten worden ist. Man erkennt einen Affinitätsunterschied un einen Faktor 2000.

B) Konjugation des monoklonalen Antikörpers mit $\beta$-Galactosidase

Gereinigte monoklonale Antikörper oder hieraus erhaltene Fab-Fragmente werden mit Maleimido-hexanoyl-hydroxysuccinimid (MHS) umgesetzt. Die MHS-modifizierten Antikörper oder Antikörper-Fragmente werden mit $\beta$-Galactosidase umgesetzt. Das Reaktionsgemisch wird über Gelfiltration (Sephacryl$^R$ S 400) aufgetrennt. Es wurde das Konjugat verwendet, das im Molekulargewichts-Bereich von 700 000 bis 20 000 000 D liegt.

C) Nachweis von Tetrajod-Thyronin

Mikrotiterplatten werden analog Abschnitt A) mit Tetrajod-Thyronin-t-BOC-Immunglobulin (1 $\mu$g/ml) beschichtet. Serumproben (50 $\mu$l) mit 0, 6, 25, 50, 125 und 247 $\mu$g/l Tetrajod-Thyronin (zur Herstellung dieser Serumproben wird TBG-freies Standardserum, Boehringer Mannheim, verwendet) werden in die Vertiefungen gefüllt und mit 150 $\mu$l Antikörper-$\beta$-Galactosidase-Konjugat (20 mU/ml) in PBS versetzt. Nach einstündiger Inkubation bei Raumtemperatur wird der Überstand entfernt, die Mikrotiter-Platte gewaschen und mit 200 $\mu$l Substrat (1 mmol Chlorphenolrot-galactosid, hergestellt gemäß DE-A-33 45 748) gefüllt. Die hieraus gewonnene Eichtabelle wird im folgenden widergegeben:

| $C_{T4}(\mu g/l)$ | mE |
|---|---|
| 0 | 620 |
| 6 | 596 |
| 25 | 519 |
| 50 | 433 |
| 125 | 320 |
| 247 | 225 |

Anhand dieser Eichtabelle können Serum-Proben mit unbekanntem T4-Gehalt bestimmt werden.

Beispiel 3

Teststreifen mit niedrigaffinem Antikörper zur Bestimmung von Theophyllin

Mit Hilfe des Immunogens Theophyllin-8-carboxypropyl-Edestin werden in bekannter Weise monoklonale Antikörper hergestellt.

Die Antikörper werden selektiert durch Kompetitionsversuche:

Je 10 $\mu$l Antikörper-Lösung und 10 $\mu$l von einer Theophyllin-Konzentrationsreihe ($5 \times 10^{-4}$ M bis $6 \times 10^{-8}$ M Theophyllin in PBS) werden zwei Stunden in einer Theophyllin-8-carboxypropyl-Kaninchen-IgG beschichteten Mikrotiter-Platte inkubiert. Der Ansatz wird dann aus der Mikrotiter-Platte entfernt, die Platte gewaschen und die an das Polyhapten gebundenen Antikörper wie in Beispiel 1 B) sichtbar gemacht.

Es wurden Antikörper ausgewählt, deren Bindung an die Polyhapten-beschichtete Platte nur mit großen Mengen Theophyllin ($>2 \times 10^{-5}$ M) kompetiert werden konnte.

Die vollständigen Antikörper bzw. die Antikörper-Fragmente werden wie in Beispiel 2 unter B) beschrieben mit $\beta$-Galactosidase verknüpft.

Herstellung der festen Trägerschichten

A) Konjugatträger

Filterpapier (Flüssigkeitsaufnahme etwa 10 $\mu$l/cm$^2$) wird mit einer Lösung getränkt, die
100 U/ml Anti-Theophyllin-8-carboxypropyl-Edestin Antikörper
1 % Rinderserum-Albumin
1 mmol Magnesiumchlorid und
25 mmol Hepes/Natriumhydroxid, pH 7,2
enthält. Anschließend wird getrocknet.

B) Polyhapten-Matrix

Auf eine Nitrozellulosemembran (0,2 $\mu$ Porengröße) wird das Hapten aufgebracht. Hierzu wird die Membran 12 bis 16 Stunden mit Polyhapten-Lösung (3 mg/ml Polyhapten in PBS) inkubiert. Danach wird zwei Stunden mit 1 mg/ml Rinderserumalbumin nachbeladen und 30 Minuten mit 0,05 % Triton$^R$ X-100 in PBS gewaschen.

C) Substratträger

Filterpapier (Flüssigkeitsaufnahme etwa 10 $\mu$l/cm$^2$) wird mit einer Lösung getränkt, die
0,4 % Tween 20
3 mM Chlorphenolrot-galactosid
25 mM Hepes/Natriumhydroxid, pH 7,2
enthält wird in üblicher Weise hergestellt.

D) Teststreifen

Mit diesen Schichten wird ein Teststreifen gemäß Abb. 4 aufgebaut. Zur Plasmaabtrennung dient ein Glasfaservlies gemäß DE-A 30 39 579.

E) Messung

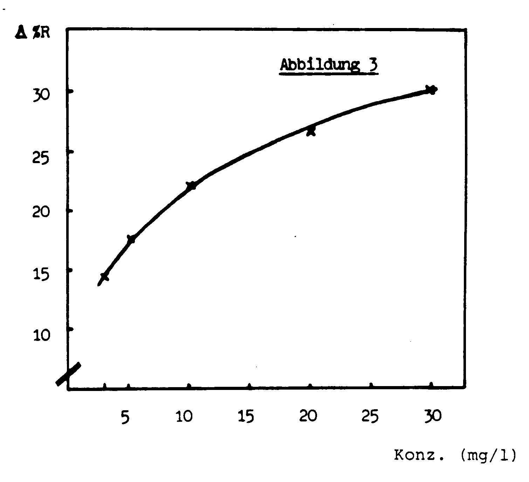

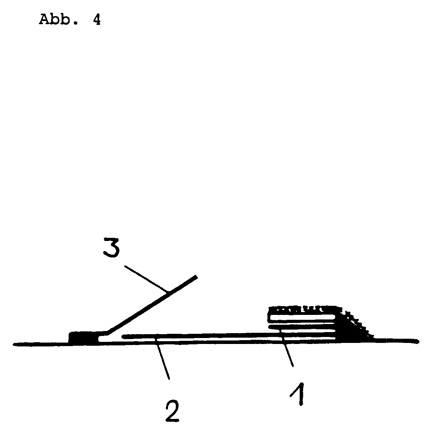

Die Probe (30 $\mu$l Vollblut, Serum oder ähnliches) wird auf das Glasfaservlies aufgegeben und diffundiert zum Konjugat auf dem Träger 1. Dieses wird dabei angelöst und tritt mit dem in der Probe vorhandenen Analyten in Wechselwirkung. Das Lösungsgemisch gelangt auf die Polyhapten-Membran 2 und wird hierüber chromatographiert. Danach wird die Klappe mit dem Substratträger 3 geschlossen. Der nicht von der Hapten-Matrix am Anfang der Matrix zurückgehaltene Teil des Lösungsgemisches kommt in Kontakt mit dem Substrat. Die Enzym-Substrat-Reaktion findet statt. Die Farbentwicklung wird nach 20 Sekunden reflektionsphotometrisch am Ende der Polyhaptenmatrix bestimmt ($\Delta$ % R = Änderung der Reflektion in % des eingestrahlten Lichtes nach 20 Sekunden). Anhand von Proben mit bekannter Theophyllin-Konzentration wird die in Abb. 3 gezeigte Eichkurve ermittelt, die den gesamten therapeutisch relevanten Bereich abdeckt.

Mit Hilfe dieser Eichkurve kann die Theophyllin-Konzentration in unbekannten Proben ermittelt werden.

Beispiel 4

Bestimmung von Phenytoin mittels niedrigaffinem Antikörper

A) Herstellung der niedrigaffinen Antikörper

Die Antikörper werden analog Beispiel 1 A) durch Immunisieren von Schafen mit Diphenylhydantoin-Valeroylsäure-Rinderserum-Albumin hergestellt. Tests zur Kompetitierbarkeit werden analog Beispiel 3 A) durchgeführt. Sie ergaben, daß eine relativ hohe Konzentration ($6 \times 10^{-6}$ M) Phenytoin zur Kompetition vom Polyhapten notwendig war (Antikörperkonzentration: ca. $5 \times 10^{-8}$ M, bestimmt durch Absorption bei 280 nm). Die Antiseren werden daher nicht weiter fraktioniert.

B) Herstellung des Testelements

Das Bestimmungsverfahren wird nach der in EP-A-073 513 beschriebenen Methode durchgeführt. Es wird das dort in den Figuren 1 a und 1 b beschriebene Einsatzelement für einen Zentrifugalanalysenautomat verwendet. Dieses Einsatzelement enthält sieben miteinander verbundene Kammern, die teilweise verschiedene Vliese enthalten und nacheinander unter dem Einfluß der Zentrifugalkraft dosiert werden. Die Vliese sind mit verschiedenen Tränklösungen imprägniert.

Folgende Vliese und Tränklösungen für die Vliese werden angewendet:

Vlies 1:          Filterpapier
Tränklösung:   3 % Netzmittel (Tween 20)
Vlies 2:          Filterpapier
Tränklösung:   100 mmol Natriumphosphatpuffer, pH 7,2
                      5 mmol EDTA
                      1 % Rinderserumalbumin
                      0,75 % Netzmittel (Tween 20)
Vlies 3:          Filterpapier
Tränklösung:   Anti-Phenytoin Antikörper vom Schaf, hergestellt nach A),
                      markiert mit $\beta$-Galactosidase, 100 mU/ml Aktivität, bestimmt mit o-Nitrophenyl-galactosid als Substrat,
                      1 % Rinderserumalbumin,
                      4 mmol Magnesium-Aspartat,
                      50 mmol Hepes-Puffer, pH 7,2
Vlies 4:          Filterpapier
Tränklösung:   2 $\mu$g/ml Kaninchen-Immunglobulin G, das mit Diphenylhedantoin-Valeroylsäure derivatisiert worden ist,
                      4 $\mu$g/ml Schaf-anti-Kaninchen-Fc Antikörper,
                      hergestellt nach DE-A-34 46 636
Vlies 5:          Filterpapier
Tränklösung:   15 mmol Chlorphenolrotgalactosid, hergestellt nach DE-A-33 45 748

Die Kammern der Einsatzelemente werden wie folgt belegt:

Kammer 1:    1 Vlies 1
Kammer 2:    leer
Kammer 3:    1 Vlies 2
Kammer 4:    1 Vlies 3
Kammer 5:    1 Vlies 4
Kammer 6:    2 Vliese 5
Kammer 7:    Meßküvette

C) Durchführung der Messung

Das als Probelösung verwendete Serum wird 1:100 mit 0,9 % Natriumchlorid-Lösung verdünnt. 60 $\mu$l der so erhaltenen Lösung werden in die Probenauftragskammer des Einsatzelementes pipettiert und dann folgendes Zentrifugationsprogramm durchgeführt:
25 Sekunden 250 rpm

Ablösung vom Detergenz, Puffer und Konjugat; Start der 1. Inkubation
20 Sekunden 2000 rpm
300 Sekunden 600 rpm
Inkubation von Probe und anti-Phenytoin-Antikörper-Konjugat
300 Sekunden 0 rpm
Inkubation mit trägerfixiertem gespacertem Phenytoin
15 Sekunden 2000 rpm
Beendigung der 2. Inkubation
15 Sekunden 0 rpm
5 Sekunden 100 rpm
Transport der Flüssigkeit in Küvetten
50 Sekunden 720 rpm
Messung bei 578 nm.

Mit dem oben beschriebenen Zentrifugationsprogramm wird zuerst von Vlies 1 das Netzmittel zur Erleichterung des Flüssigkeitstransportes abgelöst. Anschließend werden Puffer und Konjugatvlies eingeweicht und die darauf befindlichen Bestandteile gelöst. Nach 300 Sekunden Inkubation in der ersten Ventilkammer, in der sich Phenytoin mit dem anti-Phenytoin-Antikörper-Enzym-Konjugat verbindet, wird das Gleichgewicht im nächsten Schritt in den 300 Sekunden mit dem in gegenüber der Probe im Überschuß vorhandenen gespacerten und fixierten Phenytoin eingestellt. Die Lösung wird dann auf das Substrat-Vlies transportiert. Das Substrat wird dabei abgelöst. Das gesamte Flüssigkeitsgemisch gelangt in die Küvette, wo die Extinktionszunahme während 50 Sekunden verfolgt wird.

Mit Hilfe von Proben, die eine bekannte Menge Phenytoin enthalten, wird nach dem vorstehend beschriebenen Verfahren eine Eichkurve erstellt. Anhand dieser Eichkurve können Proben mit unbekanntem Phenytoin-Gehalt in entsprechender Weise gemessen werden.

**Patentansprüche**

1. Verfahren zum Nachweis eines Analyten, dadurch gekennzeichnet, daß
   - die den Analyten enthaltende Probe entweder
     mit einem markierten Bindungspartner und dem immobilisierten Analyten oder einem immobilisierten Analyt-Analogon
     oder
     mit dem markierten Analyten oder Analyt-Analogon und einem immobilisierten Bindungspartner inkubiert wird,
   - der Bindungspartner gegenüber dem immobilisierten bzw. markierten Analyten oder Analyt- Analogon eine höhere Affinität aufweist als gegenüber dem freien Analyten und
   - der markierte Bindungspartner bzw. der markierte Analyt oder das markierte Analyt-Analogon im Unterschuß verglichen mit der Konzentration des nachzuweisenden Analyten vorliegen.

2. Verfahren gemäß Anspruch 1, dadurch gekennzeichnet, daß der Analyt über ein Brückenglied an den unlöslichen Träger bzw. an das Markierungsmittel gebunden ist und daß der Bindungspartner eine höhere Affinität gegenüber der Kombination Analyt-Brückenglied als gegenüber dem freien Analyt aufweist.

3. Verfahren gemäß Anspruch 1, dadurch gekennzeichnet, daß als Analyt-Analogon eine Verbindung eingesetzt wird, die eine strukturelle Verwandtschaft mit dem Analyten aufweist und der Bindungspartner eine höhere Affinität zu dem Analyt-Analogon als zu dem zu bestimmenden Analyten besitzt.

4. Verfahren gemäß Anspruch 1, dadurch gekennzeichnet, daß das Analyt-Analogon eine charakterisierte Teilstruktur oder Teilsequenz des zu bestimmenden Analyten darstellt und der Bindungspartner eine höhere Affinität zu dieser Teilstruktur oder Teilsequenz als zu dem zu bestimmenden Analyten aufweist.

5. Verfahren gemäß einem der Ansprüche 1 bis 4, dadurch gekennzeichnet, daß das Konzentrationsverhältnis von markiertem Bindungspartner bzw. markiertem Analyten oder Analyt-Analogon zu dem nachzuweisenden Analyten bis 1:10000 beträgt.

6. Mittel zum Nachweis eines Analyten nach einem Verfahren gemäß einem der Ansprüche 1 bis 5,

EP 0 254 929 B1

dadurch gekennzeichnet, daß es

entweder

den immobilisierten Analyten oder ein immobilisiertes Analyt-Analogon und einen markierten Bindungspartner

oder

den markierten Analyten oder ein markiertes Analyt-Analogon und einen immobilisierten Bindungspartner

sowie gegebenenfalls ein geeignetes Puffersystem und weitere zusätzliche, überlicherweise verwendete Hilfsstoffe enthält, wobei der Bindungspartner gegenüber dem immobilisierten bzw. markierten Analyten oder Analyt-Analogon eine höhere Affinität aufweist als gegenüber dem freien Analyten.

7.  Mittel gemäß Anspruch 6, dadurch gekennzeichnet, daß es aus einer oder mehreren Lösungen, einem oder mehreren Trägerschichten, einer oder mehreren Filmschichten oder/und einem oder mehreren Pulvergemischen, Reagenztabletten oder Lyophilisaten besteht.

8.  Mittel gemäß Anspruch 6 oder 7, dadurch gekennzeichnet, daß es aus mindestens drei Komponenten besteht, von denen die eine den markierten Bindungspartner bzw. den markierten Analyten oder das markierte Analyt-Analogon, die andere den immobilisierten Analyten oder das immobilisierte Analyt-Analogon bzw. den immobilisierten Bindungspartner und die dritte die für den Nachweis des Markierungsmittels erforderlichen Substanzen enthält, wobei in jeder Komponente gegebenenfalls erforderliche weitere Hilfsstoffe vorhanden sind.

**Claims**

1.  Process for the detection of an analyte, characterised in that the sample containing the analyte is incubated either with a labelled binding partner and the immobilised analyte or an immobilised analyte analogue or with the labelled analyte or analyte analogue and an immobilised binding partner, the binding partner displays towards the immobilised or labelled analyte or analyte analogue a higher affinity than towards the free analyte and the labelled binding partner or the labelled analyte or the labelled analyte analogue is present in insufficiency compared with the concentration of the analyte to be detected.

2.  Process according to claim 1, characterised in that the analyte is bound via a bridge member to the insoluble carrier or to the labelling agent and that the binding partner displays a higher affinity towards the combination analyte-bridge member than towards the free analyte.

3.  Process according to claim 1, characterised in that, as analyte analogue, a compound is used which displays a structural relationship with the analyte and the binding partner possesses a higher affinity to the analyte analogue than to the analyte to be determined.

4.  Process according to claim 1, characterised in that the analyte analogue represents a characterised part structure or part sequence of the analyte to be determined and the binding partner displays a higher affinity to this part structure or part sequence than to the analyte to be determined.

5.  Process according to one of claims 1 to 4, characterised in that the concentration ratio of labelled binding partner or labelled analyte or analyte analogue to the analyte to be detected amounts to up to 1:10000.

6.  Agent for the detection of an analyte by a process according to one of claims 1 to 5, characterised in that it contains either the immobilised analyte or an immobilised analyte analogue and a labelled binding partner or the labelled analyte or a labelled analyte analogue and an immobilised binding partner, as well as possibly a suitable buffer system and further additional adjuvants usually employed, whereby the binding partner displays towards the immobilised or labelled analyte or analyte analogue a higher affinity than towards the free analyte.

7.  Agent according to claim 6, characterised in that it consists of one or more solutions, one or more carrier layers, one or more film layers and/or one or more powder mixtures, reagent tablets or lyophilisates.

11

**8.** Agent according to claim 6 or 7, characterised in that it consists of at least three components of which one contains the labelled binding partner or the labelled analyte or the labelled analyte analogue, another the immobilised analyte or the immobilised analyte analogue or the immobilised binding partner and the third the substances necessary for the detection of the labelling agent, whereby in each component are present further adjuvants possibly necessary.

## Revendications

**1.** Procédé pour la détection d'un corps à analyser caractérisé en ce que :
- l'échantillon contenant le corps à analyser est placé en incubation soit
avec un partenaire de liaison marqué et le corps à analyser fixé ou le corps à analyser équivalent fixé
soit
avec le corps à analyser marqué ou le corps à analyser équivalent marqué et un partenaire de liaison fixé,
- le partenaire de liaison présente une meilleure affinité avec le corps à analyser marqué ou fixé ou le corps analysé équivalent fixé ou marqué qu'avec le corps analysé libre et
- le partenaire de liaison marqué, respectivement le corps à analyser marqué ou le corps à analyser équivalent marqué est insuffisant comparé à la concentration du corps à analyser que l'on cherche à détecter.

**2.** Procédé selon la revendication 1 caractérisé en ce que le corps à analyser est associé en liaison pontale à un porteur insoluble, respectivement un moyen de marquage et en ce que le partenaire de liaison présente une meilleure affinité avec la combinaison liaison pontale-corps à analyser qu'avec le corps à analyser libre.

**3.** Procédé selon la revendication 1 caractérisé en ce qu'une liaison du type corps à analyser équivalent est établie, présentant une analogie structurelle avec le corps à analyser et en ce que le partenaire de liaison présente une meilleure affinité avec le corps à analyser équivalent que celle présentée par le corps à analyser correspondant.

**4.** Procédé selon la revendication 1 caractérisé en ce que le corps à analyser équivalent présente une structure partielle ou séquence partielle caractéristique du corps à analyser correspondant et en ce que le partenaire de liaison associée présente une meilleure affinité avec cette structure partielle ou séquence partielle qu'avec le corps à analyser correspondant.

**5.** Procédé selon l'une quelconque des revendications 1 à 4 caractérisé en ce que le taux de concentration du partenaire de liaison marqué, respectivement du corps à analyser ou du corps à analyser équivalent marqué, au corps à analyser que l'on cherche à détecter est de 1 : 10.000.

**6.** Moyen pour la détection d'un corps à analyser suivant le procédé selon l'une quelconque des revendications 1 à 5 caractérisé en ce qu'il comprend :
soit
le corps à analyser fixé ou un corps à analyser équivalent fixé et un partenaire de liaison marqué
soit
le corps à analyser marqué ou un corps à analyser équivalent marqué et un partenaire de liaison fixé, de même qu'éventuellement un système de tampon approprié ainsi que des produits auxiliaires utilisés de la manière habituelle par lequel le partenaire de liaison présente une meilleure affinité avec le corps à analyser ou le corps à analyser équivalent marqué, respectivement fixé, qu'avec le corps à analyser libre.

**7.** Moyen selon la revendication 6 caractérisé en ce qu'il est constitué d'une ou plusieurs solutions, d'une ou plusieurs couches supports, d'une ou plusieurs couches minces et/ou un ou plusieurs mélanges poudreux, d'un comprimé réactif ou lyophilisat.

**8.** Moyen selon la revendication 6 ou 7 caractérisé en ce qu'il comprend au moins trois composants à partir desquels, premièrement des partenaires de liaison marqué, respectivement le corps à analyser marqué, ou le corps à analyser équivalent marqué, deuxièmement le corps à analyser fixé ou le corps à analyser équivalent fixé, respectivement le partenaire de liaison fixé, et troisièmement les substances nécessaires à la détection du moyen de marquage, ainsi que les produits auxiliaires éventuellement nécessaires, sont présents dans chaque composant.

Abbildung 1

Abbildung 3

Abb. 4